# EUROPEAN PATENT APPLICATION

(11) **EP 2 692 361 A1**
(43) Date of publication of application: **05.02.2014**
(21) Application number: 12178783.2
(22) Date of filing: 01.08.2012
(51) Int. Cl.: A61K 47/48

(54) **Peptides comprising a short-chain polyethylene glycol moiety**

(71) Applicant: PEPTISYNTHA SA, 1120 Brussels (BE)
(72) Inventor: Jeannin, Laurent, 1090 Brussels (BE); Moussa, Wafa, 06300 Nice (FR); Bousmanne, Martin, 1120 Brussels (BE)
(74) Representative: Mross, Stefan P.M.

(57) **Abstract**

Described are compounds comprising a peptide moiety linked through a linkage to a short-chain polyethylene glycol moiety, more particularly compounds wherein the peptide moiety comprises a self-assembling peptide sequence, and compositions comprising these compounds. Further, methods for the preparation of the present compounds comprising a peptide moiety linked through a linkage to a short-chain polyethylene glycol moiety are disclosed.

## Description

The present invention relates to compounds comprising a peptide moiety linked through a linkage to a short-chain polyethylene glycol moiety, more particularly it relates to compounds wherein the peptide moiety comprises a self-assembling peptide sequence, and to compositions comprising these compounds. Further, the invention relates to methods for the preparation of the present compounds comprising a peptide moiety linked through a linkage to a short-chain polyethylene glycol moiety.

Self-assembling peptides comprising alternating hydrophobic and hydrophilic amino acids that self-assemble into a macroscopic structure have been reported. D.G. Osterman et al. described peptides designed to form amphiphilic β-strand or β-sheet structures (Journal of Cellular Biochemistry, vol. 29, p. 57-72, 1985). Self-assembling peptides are able to form hydrogels which can serve as matrix for various cell-based applications.

Despite the availability of peptide hydrogels, there is a continuous need for improved hydrogels providing new, or improved, applications in, for example, the fields of drug delivery or cell and tissue culture.

Accordingly, it is an object of the present invention, amongst other objects, to provide compounds that can be used in the preparation of a hydrogel suitable for cell and tissue culture with, for example, improved cell adhesion and cell growth.

Further, it is an object of the present invention, amongst other objects, to provide compounds that can be used for providing a hydrogel mimicking the extracellular matrix (ECM) and/or being more biocompatible and/or being less toxic.

Furthermore, it is an object of the present invention, amongst other objects, to provide compounds that can be used for providing a hydrogel facilitating, for example, the harvesting of cultured cells in a more economical and practical way.

Still further, it is an object of the present invention, amongst other objects, to provide compounds with an improved aqueous solubility and/or an improved wettability.

Yet another object of the present invention, amongst other objects, is to provide compounds with improved properties that may be suitable as a vehicle for the formulation of cells, e.g. in cell therapy, and/or that may be applicable for parenteral application. Furthermore, the compounds might show improved viscosity and/or improved rheological behavior.

The above objects, amongst other objects, are met at least partially, if not completely, by one or more embodiments of this invention.

Accordingly, one embodiment of the invention relates to a compound comprising a peptide moiety linked through a linkage to a short-chain polyethylene glycol moiety.

### Brief description of the drawings:

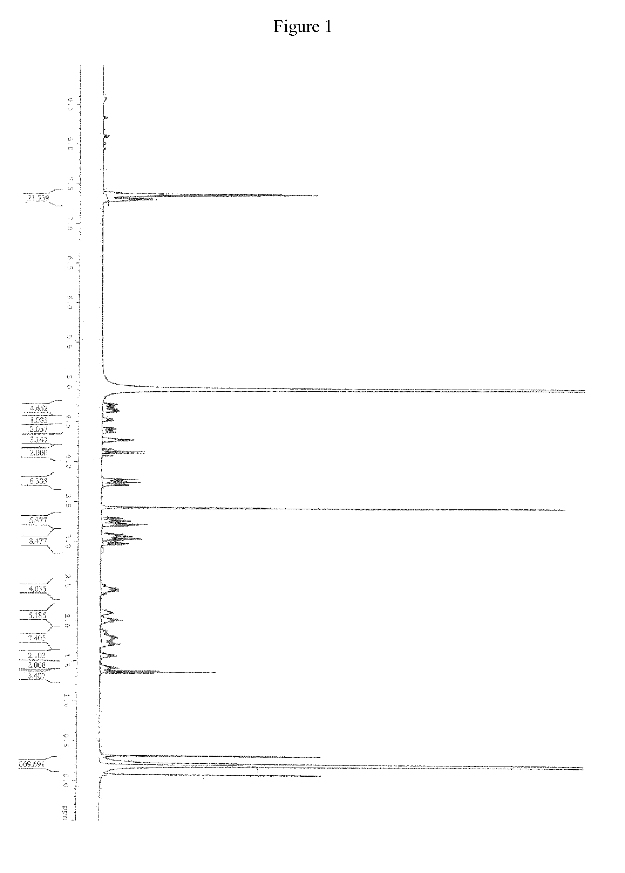

Figure 1 shows the ¹H NMR spectrum of the product according to example 4 (SEQ ID NO:100).

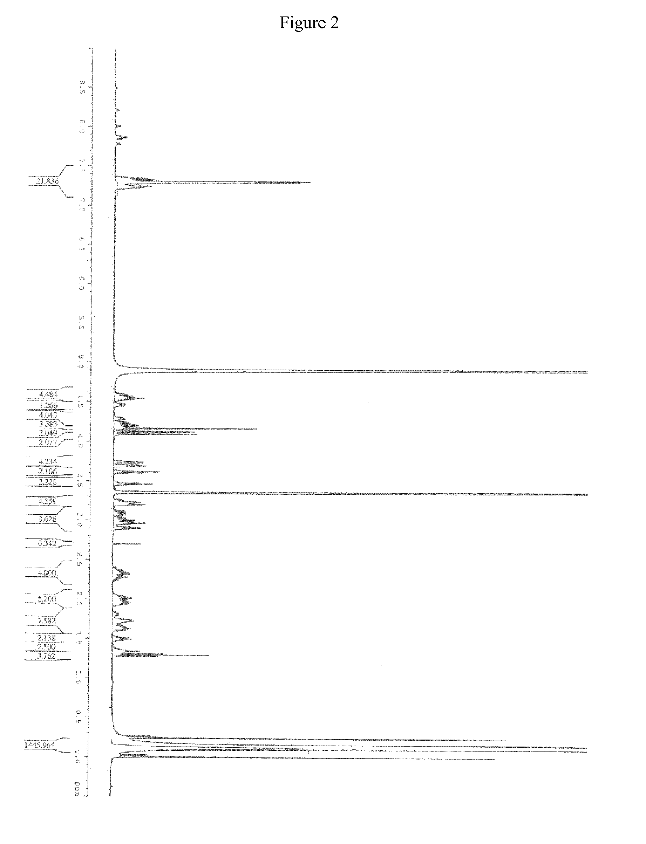

Figure 2 shows the ¹H NMR spectrum of the product according to example 7 (SEQ ID NO:103).

As used herein, the term "short-chain polyethylene glycol" refers to an oligomer of oxyethylene according to the formula :

HOCH₂(CH₂-O-CH₂-)ₙCH₂O-

wherein n is 0 to 24. As used herein, the term "oxyethylene" refers to the moiety -OCH₂CH₂-. Thus, the short-chain polyethylene glycol moiety comprises equal to or less than 25 oxyethylene moieties. In a preferred embodiment the short-chain polyethylene glycol moiety comprises equal to or less than 10 oxyethylene moieties, more preferably equal to or less than 5 oxyethylene moieties. Most preferably, the short-chain polyethylene glycol moiety comprises from 1 to 5 oxyethylene moieties.

In another preferred embodiment the short-chain polyethylene glycol moiety comprises one or more functional groups.

As used herein, "functional group" is intended to denote atoms or small groups of atoms that have special chemical properties and which define the chemistry of an organic compound. Examples of functional groups include alkenyl, alkynyl, (hetero)aryl, hydroxyl, amine, ammonium, carbonyl, carboxyl, phosphate, sulphate, carboxamide, carbonate, carbamate, urea, ester, nitrate, nitro and nitrile. The functional group can be linked to the short-chain polyethylene glycol moiety directly, i.e. through a chemical bond ; or indirectly through a linking group. Suitable linking groups are given below. More preferred are functional groups that can be protonated or deprotonated under physiological conditions. As used herein, the term "physiological conditions" is intended to denote conditions of the external or internal milieu that may occur in nature for organisms or cell cultures. A temperature range of 20-40 °C and pH of 6-8 are examples of physiological conditions as used herein. Most preferably, the functional group is selected from the group consisting of NH₂ and COOH.

As used herein, "linkage" is intended to denote any means of covalently connecting two different moieties within a compound of the invention. In a preferred embodiment such linkage can be a direct linkage, i.e. a covalent bond between the atoms of the peptide moiety and the atoms of the short-chain polyethylene glycol moiety. Alternatively, the linkage can be indirect, i.e., through a linking group. Examples of suitable linking groups are a linear or branched alkylidene, especially a polymethylene group comprising 1 to 10 carbon atoms ;
a thioether linkage, preferably according to the formula:

― [C(O)]_{z}―(CH₂)_{w}―S―(CH₂)ₓ―[C(O)]_{y}―

wherein w and x independently are 0-10, preferably 1, 2, 3 or 4 ; y and z independently are 0 or 1 ;
an amino linkage, preferably according to the formula:

― [C(O)]_{z}―(CH₂)_{w}―NH―(CH₂)ₓ―[C(O)]_{y}―

wherein w and x independently are 0-10, preferably 1, 2, 3 or 4 ; y and z independently are 0 or 1 ;
an amido linkage, preferably according to the formula:

-C(O)-NH-(CH₂)ₓ―[C(O)]_{y}-

wherein x is 0-10, preferably x is 1, 2, 3, 4 or 5, more preferably x is 1, 2, 3 or 4 ; y is 0 or 1 ;
an ester linkage, preferably according to the formula :

-C(O)-O-(CH₂)ₓ―[C(O)]_{y}-

wherein x is 1-10, preferably x is 1, 2, 3 or 4 ; y is 0 or 1;
or an ether linkage, preferably according to the formula :

― [C(O)]_{z}―(CH₂)_{w}―O―(CH₂)ₓ―[C(O)]_{y}―

wherein w and x independently are 0-10, preferably 1, 2, 3 or 4 ; y and z independently are 0 or 1.

In a more preferred embodiment, the linkage is -C(O)CH₂OCH₂C(O)-, -C(O)CH₂CH₂C(O)- or -C(O)CH₂CH₂CH₂C(O)-.

As used herein, the term "peptide moiety" comprises peptides and peptide analogues. Peptide analogues comprise natural amino acids and non-natural amino acids. They can also comprise modifications such as ester or amide formation on the C-terminus or N-terminus, respectively. An example for a modification on the C-terminus is the introduction of an ethyl ester which can be accomplished by methods known in the art. All amino acids can be either the L-or D- isomer. The peptides or peptide analogues can also comprise amino acid mimetics that function in a manner similar to the naturally occurring amino acids. The peptides may also be formed from amino acids analogues that have modified R groups or modified peptide backbones. Peptide analogues usually include at least one bond in the peptide sequence which is different from an amide bond, such as urethane, urea, ester or thioester bond. Peptides or peptide analogues according to the present invention can be linear, cyclic or branched and are preferably linear.

As used herein, the term "amino acid" (Xaa) is intended to denote any compound comprising at least one NR₁R₂ group, preferably at least one NH₂ group, and at least one carboxyl group. The amino acids of this invention can be naturally occurring or synthetic. The natural amino acids, with exception of glycine, contain a chiral carbon atom. Unless otherwise specifically indicated, the compounds containing natural amino acids with the L-configuration are preferred. The amino acids can be selected from, for example, β-alanine, γ―aminobutyric acid, 5-aminovaleric acid, glycine, phenylglycine, homoarginine, alanine, valine, norvaline, leucine, norleucine, isoleucine, serine, isoserine, homoserine, threonine, allothreonine, methionine, ethionine, glutamic acid, aspartic acid, asparagine, cysteine, cystine, phenylalanine, tyrosine, tryptophan, lysine, hydroxylysine, arginine, histidine, ornithine, glutamine, citrulline, proline, and 4-hydroxyproline. Amino acid residues are abbreviated as follows throughout the application : Alanine is Ala or A ; β-Alanine is β―Ala ; γ―aminobutyric acid is GABA ; 5-aminovaleric acid is Ava ; Arginine is Arg or R ; Homoarginine is Har or hR ; Alanine is Ala or A ; Asparagine is Asn or N ; Aspartic acid is Asp or D ; Cysteine is Cys or C ; Glutamic acid is Glu or E ; Glutamine is Gln or Q ; Glycine is Gly or G ; Histidine is His or H ; Homoserine is Hse ; Hydroxylysine is Hyl ; Isoleucine is Ile or I ; Leucine is Leu or L ; Lysine is Lys or K ; Methionine is Met or M ; Norleucine is Nle ; Ornithine is Orn ; Phenylalanine is Phe or F ; Proline is Pro or P ; 4-Hydroxyproline is Hyp or O ; Serine is Ser or S ; Threonine is Thr or T ; Tryptophan is Trp or W ; Tyrosine is Tyr or Y ; Valine is Val or V.

In a preferred embodiment the peptide moiety is able to self-assemble in a β-sheet, a coiled coil α-helix structure, a peptide triple helix structure, or combinations thereof. Self-assembling amino acid sequences capable of assembling into a β-sheet are more preferred.

According to another preferred embodiment of the present invention, the peptide moiety is an octapeptide moiety comprising alternating hydrophobic and charged amino acids. Hydrophobic amino acids are often selected from the group consisting of Phenylalanine (Phe or F), Tryptophan (Trp or W), Tyrosine (Tyr or Y), Isoleucine (Ile or I), Alanine (Ala or A), Leucine (Leu or L), Valine (Val or V), and Norleucine (Nle) ; in particular from Phenylalanine (Phe or F), Tryptophan (Trp or W), Tyrosine (Tyr or Y), Isoleucine (Ile or I), and Norleucine (Nle). Charged amino acids are usually selected from the group consisting of Arginine (Arg or R), Aspartic acid (Asp or D), Glutamic acid (Glu or E), Lysine (Lys or K), and Histidine (His or H) ; particularly from Arginine (Arg or R), Aspartic acid (Asp or D), Glutamic acid (Glu or E), and Lysine (Lys or K).

According to another preferred embodiment of the present invention the octapeptides comprise one type of hydrophobic amino acid and two types of charged amino acids. Especially suitable octapeptides are formed by the combination of two sequences chosen independently from the group consisting of FEFE (SEQ ID NO:1), FEFK (SEQ ID NO:2), FEFD (SEQ ID NO:3), FEFR (SEQ ID NO:4), FRFR (SEQ ID NO:5), FRFK (SEQ ID NO:6), FRFE (SEQ ID NO:7), FRFD (SEQ ID NO:8), FKFE (SEQ ID NO:9), FKFK (SEQ ID NO:10), FKFR (SEQ ID NO:11), FKFD (SEQ ID NO:12), FDFD (SEQ ID NO:13), FDFE (SEQ ID NO:14), FDFR (SEQ ID NO:15), FDFK (SEQ ID NO:16), WEWE (SEQ ID NO:17), WKWK (SEQ ID NO:18), WRWR (SEQ ID NO:19), WEWK (SEQ ID NO:20), WKWE (SEQ ID NO:21), WEWR (SEQ ID NO:22), WRWE (SEQ ID NO:23), WKWR (SEQ ID NO:24), WRWK (SEQ ID NO:25), WDWD (SEQ ID NO:26), WDWE (SEQ ID NO:27), WEWD (SEQ ID NO:28), WDWK (SEQ ID NO:29), WKWD (SEQ ID NO:30), WDWR (SEQ ID NO:31), WRWD (SEQ ID NO:32), IEIE (SEQ ID NO:33), IEIK (SEQ ID NO:34), IRIR (SEQ ID NO:35), IKIK (SEQ ID NO:36), IKIE (SEQ ID NO:37), IEIR (SEQ ID NO:38), IRIE (SEQ ID NO:39), IKIR (SEQ ID NO:40), IRIK (SEQ ID NO:41), IDID (SEQ ID NO:42), IDFE (SEQ ID NO:43), IEID (SEQ ID NO:44), IDIK (SEQ ID NO:45), IKID (SEQ ID NO:46), IDIR (SEQ ID NO:47), IRID (SEQ ID NO:48), YEYE (SEQ ID NO:49), YKYK (SEQ ID NO:50), YRYR (SEQ ID NO:51), YEYK (SEQ ID NO:52), YKYE (SEQ ID NO:53), YEYR (SEQ ID NO:54), YRYE (SEQ ID NO:55), YKYR (SEQ ID NO:56), YRYK (SEQ ID NO:57), YDYD (SEQ ID NO:58), YDYE (SEQ ID NO:59), YEYD (SEQ ID NO:60), YDYK (SEQ ID NO:61), YKYD (SEQ ID NO:62), YDYR (SEQ ID NO:63), YRYD (SEQ ID NO:64), Nle-E-Nle-E (SEQ ID NO:65), Nle-K-Nle-K (SEQ ID NO:66), Nle-R-Nle-R (SEQ ID NO:67), Nle-E-Nle-K (SEQ ID NO:68), Nle-K-Nle-E (SEQ ID NO:69), Nle-E-Nle-R (SEQ ID NO:70), Nle-R-Nle-E (SEQ ID NO:71), Nle-K-Nle-R (SEQ ID NO:72), Nle-R-Nle-K (SEQ ID NO:73), Nle-D-Nle-D (SEQ ID NO:74), Nle-D-Nle-E (SEQ ID NO:75), Nle-E-Nle-D (SEQ ID NO:76), Nle-D-Nle-K (SEQ ID NO:77), Nle-K-Nle-D (SEQ ID NO:78), Nle-D-Nle-R (SEQ ID NO:79), and Nle-R-Nle-D (SEQ ID NO:80). The two sequences can be the same or different, especially the same.

According to yet another preferred embodiment of the present invention the octapeptide moiety might for instance be selected from the group consisting of FEFKFEFK (SEQ ID NO:81), FEFEFKFK (SEQ ID NO:82), FDFKFDFK (SEQ ID NO:83), FDFDFKFK (SEQ ID NO:84), FEFRFEFR (SEQ ID NO:85), FEFEFRFR (SEQ ID NO:86), YDYKYDYK (SEQ ID NO:87), YDYDYKYK (SEQ ID NO:88), YEYRYEYR (SEQ ID NO:89), YEYKYEYK (SEQ ID NO:90), YEYEYKYK (SEQ ID NO:91), WEWKWEWK (SEQ ID NO:92), WEWEWKWK (SEQ ID NO:93), WDWKWDWK (SEQ ID NO:94), WDWDWKWK (SEQ ID NO:95). Most preferably the amino sequence is FEFKFEFK (SEQ ID NO:81).

In yet another preferred embodiment the peptide moiety is linked through a linkage to the short-chain polyethylene glycol moiety at the terminus of the peptide moiety. More preferably, it is linked through a linkage at the C-terminus of the peptide moiety.

Another embodiment of the invention relates to a composition comprising a compound according to the invention as described above and further comprising at least one solvent and/or comprising at least one additive. Examples for suitable solvents are water, ethanol, methanol, isopropanol, propanol, butanol, acetonitrile, acetone, dimethylsulfoxide, N-methylpyrrolidinone, N,N-dimethylformamide, N,N-dimethylacetamide, or mixtures thereof. Especially suitable is water or mixtures of water with at least one further solvent. Examples for suitable additives for cell culture are nutrients, antibiotics, buffers, and/or growth factors. Examples for suitable buffers are Hank's Balanced Salt Solution (HBSS) and Dulbecco's Modified Eagle Medium (DMEM).

Preferably, the composition is a pharmaceutical composition comprising a compound according to the invention. The pharmaceutical composition may be administered parenterally, for example, intravenously, intraarterially, intraperitoneally, intrathecally, intraventricularly, intraurethrally, intrasternally, intracranially, intramuscularly or subcutaneously, or they may be administered using needleless injection techniques. For such parenteral administration a sterile aqueous solution which may contain other substances, for example, enough salts or glucose to make the solution isotonic with blood may be used. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

In another preferred embodiment, the composition further comprises a further peptide. An example of a suitable further peptide is a peptide that is able to self-assemble. An especially suitable further peptide comprises the same sequence as the peptide moiety of the compound of the invention comprised in the composition.

As indicated above, the compounds comprising a peptide moiety linked through a linkage to a short-chain polyethylene glycol moiety according to the invention are capable of providing a hydrogel suitable for cell and tissue culture.

Accordingly, another embodiment of the invention are hydrogels comprising a compound or a composition according to this invention as described above.

The preparation of a hydrogel according to the invention may comprise the steps of:
a) adding at least water to a compound or to a composition according to the invention ;
b) optionally adding a further peptide that is able to self-assemble ;
c) optionally adjusting the pH and/or the ionic strength of the resulting medium, with or without the further peptide that is able to self-assemble, to form a hydrogel.

Step a) of the method for preparing a hydrogel may further comprise adding at least one further suitable solvent. Examples of suitable solvents or solvent mixtures are given above. The hydrogels thus obtained show thixotropic behaviour suitable for therapeutic and/or cell culture applications.

According to another aspect, the present invention relates to methods for preparing the present compounds comprising a peptide moiety linked through a linkage to a short-chain polyethylene glycol moiety. The short-chain polyethylene glycol moiety optionally comprising a functional group and/or the linkages according to the present invention may be coupled to the peptide moiety using techniques known to the skilled man and may proceed via compounds such as mercapto propionic acid, γ-amino butyric acid, ε-amino caproic acid, 3-aminopropionic acid, 5-amino valeric acid, 11-amino undecanoic acid, 8-amino-3,6-dioxaoctanoic acid, succinic anhydride, glutaric anhydride, diglycolic anhydride, or 1-amino-4.7.10-trioxa-13-tridecanamine. The functional groups present in the coupling partners may be suitably protected during the coupling step between the coupling partners. Protecting groups are known in the art of peptide synthesis. Examples of suitable protecting groups include carboxybenzyl (Z) or tert-butyloxycarbonyl (Boc) for an amine and esters such as a tert-butyl ester for a carboxyl group. Protection and deprotection may be performed as known in the art, for example by hydrogenation or acidic cleavage, e.g. using trifluoroacetic acid (TFA). The coupling may be facilitated by using coupling reagents. Examples of coupling reagents include 1-hydroxybenzotriazole, 1-hydroxy-7-azabenzotriazole, N,N'-dicyclohexylcarbodiimide, N,N'-diisopropylcarbodiimide and 1-ethyl-3-(3-dimethylaminopropyl)carbodiimide. Examples for solvents suitable in these reactions include acetonitrile, acetone, dimethylsulfoxide, N-methylpyrrolidinone, N,N-dimethylformamide, N,N-dimethylacetamide, or mixtures thereof.

Should the disclosure of any patents, patent applications, and publications which are incorporated herein by reference conflict with the description of the present application to the extent that it might render a term unclear, the present description shall take precedence.

The present invention is further illustrated below without limiting the scope thereto.

### Examples :

### General methods :

**HPLC** : Analyses were performed on an Agilent 1100 series HPLC using "method CH-GS-10" : Column : Merck Chromolith RP C18-e (100 mm x 4.6 mm) ; mobile phase A : water + 0.1 % TFA, mobile phase B : acetonitrile + 0.1 % TFA ; stoptime : 10.5 min ; posttime : 1.0 min ; temperature : 40 °C ; flow rate : 4.0 ml/min.

Gradient :

| | |
|---|---|
| Time (min) | % solvent B |
| 0.00 | 2.0 |
| 10.00 | 91.1 |
| 10.10 | 100.0 |
| 10.50 | 100.0 |
| 10.60 | 2.0 |

¹H NMR : Bruker AVANCE, 500 MHz ; solvent: CD₃OD + 2-3 drops TFA ; internal standard : octamethycyclotetrasiloxane.

### Example I : Z-Phe-Glu(OtBu)-Phe-Lys(Boc)-Phe-Glu(OtBu)-Phe-Lys(Boc)-OEt (SEQ ID NO:97)

50 g Z-Phe-Glu(OtBu)-Phe-Lys(Boc)-Phe-Glu(OtBu)-Phe-Lys(Boc)-OH (SEQ ID NO:96) (32 mmol) and 5.3 g caesium carbonate (16 mmol) were added to 500 ml N,N-dimethylformamide. 5.5 ml iodoethane (69 mmol) was added and the solution was stirred at 48°C for 2 h. After filtration and partial evaporation of the N,N-dimethylformamide, the concentrate was poured into 500 ml 2.5 wt.- % aqueous KHSO₄, filtered, washed with water and finally with 500 ml warm ethanol. After drying under reduced pressure at 45°C, 47 g (84 %) of a solid was obtained. The HPLC analysis of the product was carried out as follows : a sample was dissolved in 1 ml N,N- dimethylacetamide and analysed using method CH-GS-10. Product purity : 97 % of surface area (HPLC).

### Example 2 : H-Phe-Glu(OtBu)-Phe-Lys(Boc)-Phe-Glu(OtBu)-Phe-Lys(Boc)-OEt (SEQ ID NO:98)

21.6 g Z-Phe-Glu(OtBu)-Phe-Lys(Boc)-Phe-Glu(OtBu)-Phe-Lys(Boc)-OEt (SEQ ID NO:97) (13.6 mmol) was dissolved in 215 ml N,N-dimethylacetamide. After flushing the solution several times with nitrogen gas, 14.5 g Pd/SiO₂ (2 wt.- %) was added and subsequently, hydrogen gas was introduced. After stirring for 2 h, the suspension was passed through a 0.45 µm filter and the Pd/SiO₂ was washed with N,N-dimethylacetamide. The combined filtrates can be used without further purification in the subsequent step. The yield was quantitative.

### Example 3 : Boc-HNCH₂CH₂OCH₂CH₂OCH₂C(O)Phe-Glu(OtBu)-Phe-Lys(Boc)-Phe-Glu(OtBu)-Phe-Lys(Boc)-OEt (SEQ ID NO:99)

1.0 g 8-(t-Butyloxycarbonyl-amino)-3,6-dioxaoctanoic acid (Boc-O2OC-OH.DCHA) (2.25 mmol) was suspended in 50 ml CH₂Cl₂. This suspension was washed with 60 ml 5 % aqueous NaCl solution containing 0.43 g KHSO₄ (3.15 mmol), then with 60 ml 5 % aqueous NaCl solution and finally with 60 ml distilled water. After concentration in vacuo and azeotropic drying, a sample of the concentrated solution was titrated with 0.1 N aqueous NaOH solution. A N,N-dimethylacetamide solution of H-Phe-Glu(OtBu)-Phe-Lys(Boc)-Phe-Glu(OtBu)-Phe-Lys(Boc)-OEt (SEQ ID NO:98) (1.25 mmol, 20 ml) was added to the concentrated solution of neutralized Boc-020C-OH (1.2 mmol). 1-Ethyl-3-(3-dimethylaminopropyl)carbodiimide (0.2 g, 1.3 mmol) and N-hydroxybenzotriazole (0.18 g, 1.3 mmol) were added at room temperature. After HPLC control of the completion of the reaction, the reaction mixture was poured into 200 ml 2.5 % aqueous KHSO₄ solution. The resulting precipitate was washed with 25 ml distilled water. After drying in vacuo at 45°C, 1.58 g (1 mmol) of an off-white solid was obtained. The HPLC analysis of the product was carried out as follows : a sample was dissolved in 1 ml N,N-dimethylacetamide and analysed using method CH-GS-10 ; t_{R} = 8.9 ± 0.5 min.

### Example 4 : H₂NCH₂CH₂OCH₂CH₂OCH₂C(O)Phe-Glu-Phe-Lys-Phe-Glu-Phe-Lys-OEt (SEQ ID NO:100)

20 ml trifluoroacetic acid, 2 ml triisopropylsilane, 1 ml ethanol and 1 ml water were dissolved in 20 ml CH₂Cl₂. To this solution, 1.5 g (0.9 mmol) BOCHNCH₂CH₂OCH₂CH₂OCH₂C(O)Phe-Glu(OtBu)-Phe-Lys(Boc)-Phe-Glu(OtBu)-Phe-Lys(Boc)-OEt (SEQ ID NO:99) was added at room temperature. After stirring at room temperature for 1 h, the reaction mixture was concentrated in vacuo. The resulting solid was dispersed in 30 ml methyl t-butyl ether and filtered. 1.26 g of an off-white product was obtained. The HPLC analysis of the product was carried out as follows : a sample was dissolved in 1 ml N,N-dimethylacetamide and analyzed using method CH-GS-10 ; t_{R} = 4.1 ± 0.5 min. Product purity : 85 % of surface area (HPLC). The product can be purified further by preparative HPLC to obtain a purity ≥ 95 % (surface area) and additionally, can optionally be lyophilized.

### Example 5 : Synthesis of HOOC-CH₂-O-CH₂-C(O)-Phe-Glu(OtBu)-Phe-Lys(Boc)-Phe-Glu(OtBu)-Phe-Lys(Boc)-OEt (SEQ ID NO:101)

0.22 g diglycolic anhydride (1.6 mmol) was added to a solution of 35.5 g H-Phe-Glu(OtBu)-Phe-Lys(Boc)-Phe-Glu(OtBu)-Phe-Lys(Boc)-OEt (SEQ ID NO:98) in N,N-dimethylacetamide. After stirring at room temperature for at least one hour, the reaction mixture was partially concentrated under vacuum and then poured into a solution of KHSO₄ (0.43 g) in 150 ml water. The resulting precipitate was filtered, washed with water (50 ml) twice and then dried under vacuum at 45°C. After drying, an off-white product (2.39 g, 95 %) was obtained. The HPLC analysis of the product was carried out as follows : a sample was dissolved in 1 ml N,N-dimethylacetamide and analysed using method CH-GS-10 ; t_{R} = 8.3 ± 0.5 min. Product purity : 88 % of surface area (HPLC).

### Example 6 : Synthesis of HOOCCH₂OCH₂CH₂OCH₂CH₂NC(O)CH₂OCH₂C(O)-Phe-Glu(OtBu)-Phe-Lys(Boc)-Phe-Glu(OtBu)-Phe-Lys(Boc)-OEt (SEQ ID NO:102)

8.9 g HOOCCH₂OCH₂C(O)-Phe-Glu(OtBu)-Phe-Lys(Boc)-Phe-Glu(OtBu)-Phe-Lys(Boc)-OEt (SEQ ID NO:101) (5.6 mmol), 1.15 ml diisopropylethylamine (5.68 mmol) and 0.47 ml pyridine (5.68 mmol) were dissolved in a mixture of 70 ml N,N-dimethylacetamide and 20 ml dichloromethane. The reaction mixture was cooled to -5°C (solution n°1). 1.02 g 8-amino-3,6-dioxaoctanoic acid (6.19 mmol) was dispersed in 2.30 ml dichloromethane containing 1.84 g trimethylsilylacetamide (12.4 mmol) (solution n°2). Solution n°2 was stirred at room temperature and then cooled to 10°C. At -5°C, 0.7 g pivaloyl chloride was added to solution n°1. After about 5 min of further stirring, cooled solution n°2 was added to solution n°1. The reaction mixture was allowed to warm to room temperature. After controlling the completion of the reaction by HPLC, 2 ml water was added, the reaction mixture partially concentrated under vacuum and then poured into a solution of KHS04 (2.5 g) in 500 ml water. The resulting precipitate was filtered, washed with water (50 ml) twice and then dried under vacuum at 45°C. After drying, 9.17 g (94 %) of an off-white product was obtained. The HPLC analysis of the product was carried out as follows : a sample was dissolved in 1 ml N,N-dimethylacetamide and analysed using method CH-GS-10 ; t_{R} = 7.8 ± 0.5 min. Product purity : 89 % of surface area (HPLC).

### Example 7 : Synthesis of HOOCCH₂OCH₂CH₂OCH₂CH₂NC(O) CH₂OCH₂C(O)-Phe-Glu-Phe-Lys-Phe-Glu-Phe-Lys-OEt (SEQ ID NO:103)

11.44 g HOOCCH₂OCH₂CH₂OCH₂CH₂NC(O)CH₂OCH₂C(O)-Phe-Glu(OtBu)-Phe-Lys(Boc)-Phe-Glu(OtBu)-Phe-Lys(Boc)-OEt (SEQ ID NO:102) was added to 120 ml of a mixture of trifluoroacetic acid and water (95/5) under stirring. After 30 min at room temperature, the reaction mixture was partially concentrated under vaccum, diluted with dichloromethane (20 ml) and concentrated again. This step of concentrating and dilution was repeated several times. The concentrated solution was added to 1000 ml diisopropylether and a precipitate was formed. After filtration, the precipitate was washed twice with 500 ml diisopropylether and dried under vacuum at 45°C to yield 10.62 g (85 %) of an off-white solid. The HPLC analysis of the product was carried out as follows : a sample was dissolved in 1 ml N,N-dimethylacetamide and analysed using method CH-GS-10 ; t_{R} = 4.2 ± 0.5 min. Product purity : 85 % of surface area (HPLC). This crude can optionally be purified by HPLC to reach a purity ≥ 95 % (surface area) and can optionally be lyophilized.

### Example 8 : Synthesis of Z-Phe-Glu(OtBu)-Phe-Lys(Boc)-Phe-Glu(OtBu)-Phe-Lys(Boc)-NH-(CH₂)₃-O-(CH₂)₂-O-(CH₂)₂-O-(CH₂)₃-NHBoc (SEQ ID NO:104)

0.784 g Z-Phe-Glu(OtBu)-Phe-Lys(Boc)-Phe-Glu(OtBu)-Phe-Lys(Boc)-OH (SEQ ID NO:96) (0.5 mmol) and 0.165 g 1-(t-butyloxycarbonylamino)-4,7,10-trioxa-13-tridecanamine (Boc-TOTA-NH₂) (0.5 mmol) were dissolved in 8 ml N,N-dimethylacetamide at room temperature. After cooling to 0°C, 83 mg 1-ethyl-3-(3-dimethylaminopropyl)carbodiimide (0.6 mmol) and 117 mg N-hydroxybenzotriazole (0.6 mmol) were added to the solution. The solution was stirred at 0°C for an additional 3 h and then allowed to warm to room temperature. After control of the completion of the reaction by HPLC, the reaction mixture was added to a solution of KHSO₄ (0.1 g) in 80 ml water. The resulting precipitate was washed with 25 ml distilled water twice. After drying under vacuum at 45°C, 820 mg (87 %) of an off-white solid was obtained. The HPLC analysis of the product was carried out as follows : a sample was dissolved in 1 ml N,N-dimethylacetamide and analysed using method CH-GS-10 ; t_{R} = 9.2 ± 0.5 min. Product purity : 88 % of surface area (HPLC).

### Example 9 : Synthesis of H Phe-Glu(OtBu)-Phe-Lys(Boc)-Phe-Glu(OtBu)-Phe-Lys(Boc)-NH-(CH₂)₃-O-(CH₂)₂-O-(CH₂)₂-O-(CH₂)₃-NHBoc (SEQ ID NO:105).

820 mg Z-Phe-Glu(OtBu)-Phe-Lys(Boc)-Phe-Glu(OtBu)-Phe-Lys(Boc)-NH-(CH₂)₃-O-(CH₂)₂-O-(CH₂)₂-O-(CH₂)₃-NHBoc (SEQ ID NO:104) (0.4 mmol) was dissolved in 20 ml N,N- dimethylacetamide and stirred at 45°C. After flushing the solution several times with nitrogen gas, 0.47 g Pd/SiO₂ (2 wt.- %) was added. Hydrogen gas was introduced and the reaction was stirred for 2 h. The suspension was passed through a 0.45 µm filter and the Pd/SiO₂ was washed with N,N- dimethylacetamide. The combined filtrates were partially concentrated under vacuum and poured into a solution of NaHCO₃ (0.05 g) in 35 ml water. After filtration, the precipitate was washed several times with water (10 ml each). After drying under vacuum at 45°C, 490 mg (83 %) of an off-white solid was obtained. The HPLC analysis of the product was carried out as follows : a sample was dissolved in 1 ml N,N-dimethylacetamide and analysed by the method CH-GS-10 ; t_{R} = 7.2 ± 0.5 min. Product purity : 90 % of surface area (HPLC).

### Example 10 : Synthesis of H-Phe-Glu-Phe-Lys-Phe-Glu-Phe-Lys-NH-(CH₂)₃-O-(CH₂)₂-O-(CH₂)₂-O-(CH₂)₃-NH₂ (SEQ ID NO:106)

490 mg H-Phe-Glu(OtBu)-Phe-Lys(Boc)-Phe-Glu(OtBu)-Phe-Lys(Boc)-NH-(CH₂)₃-O-(CH₂)₂-O-(CH₂)₂-O-(CH₂)₃-NHBoc (SEQ ID NO:105) was added to 5 ml of a mixture of trifluoroacetic acid and water (95/5) under stirring. After 30 min stirring at room temperature, the reaction mixture is partially concentrated under vacuum, diluted with CH₂Cl₂ and concentrated again. This step of concentrating and dilution was repeated several times. The concentrated solution was then poured into 35 ml diisopropylether to obtain a precipitate which was collected. After drying the precipitate under vacuum at 45°C, 420 mg (85 %) of an off-white solid was obtained. The HPLC analysis of the product was carried out as follows : a sample was dissolved in 1 ml N,N-dimethylacetamide and analysed using method CH-GS-10 ; t_{R} = 3.5 ± 0.5 min. Product purity : 88 % of surface area (HPLC). This crude may be purified further by preparative HPLC to reach a purity of ≥ 95 % (surface area) and optionally lyophilized to obtain the trifluoroacetate salt.

### Example 11 : Hydrogel preparation

0.81 mg NaHCO₃ was added to 0.1 ml of a dimethylsulfoxide solution of 0.02 g HOOCCH₂OCH₂CH₂OCH₂CH₂NC(O)CH₂OCH₂C(O)-Phe-Glu-Phe-Lys-Phe-Glu-Phe-Lys-OEt (SEQ ID NO:103). The suspension is vigorously shaken. The suspension is diluted with 3.9 ml Hank's Balanced Salt Solution (HBSS) and the mixture was shaken for 3 min. The resulting gel was transferred to a Vivaspin® ultrafiltration spin column with a molecular weight cut-off varying from 3 to 100 kDa, and centrifuged between 12 000 and 4000 G for at least 15 min. The filtrate was discarded. The same volume of fresh culture media was added on top of the gel, and another centrifugation cycle was performed. This operation was repeated at least twice. During the last washing, fresh HBSS was replaced by fresh Dulbecco's Modified Eagle Medium (DMEM). The washed hydrogel was finally conditioned and stored at 10°C prior use.

## Claims

1. A compound comprising a peptide moiety linked through a linkage to a short-chain polyethylene glycol moiety wherein the short-chain polyethylene glycol moiety comprises equal to or less than 25 oxyethylene moieties, preferably equal to or less than 10 oxyethylene moieties, more preferably equal to or less than 5 oxyethylene moieties.

2. The compound of claim 2 wherein the linkage is a chemical bond or a linear or branched alkylidene linkage, a thioether linkage, a amino linkage, amide linkage, ester linkage or a ether linkage, preferably the linkage is -C(O)CH₂OCH₂C(O)-, -C(O)CH₂CH₂C(O)-, or -C(O)CH₂CH₂CH₂C(O)-.

3. The compound of claim 1 or 2 any wherein the short-chain polyethylene glycol moiety comprises one or more functional groups, preferably one or more functional groups that can be protonated or deprotonated under physiological conditions, particularly one or more functional groups that are selected from the group consisting of NH₂ and COOH.

4. The compound according to any one of the claims 1 to 3, wherein the peptide moiety is able to self-assemble in a β-sheet, a coiled coil α-helix structure, a peptide triple helix structure, or combinations thereof, preferably the peptide moiety is able to self-assemble in a β-sheet.

5. The compound according to any one of the claims 1 to 4, wherein the peptide moiety is an octapeptide moiety formed by the combination of two sequences chosen independently from the group consisting of FEFE (SEQ ID NO:1), FEFK (SEQ ID NO:2), FEFD (SEQ ID NO:3), FEFR (SEQ ID NO:4), FRFR (SEQ ID NO:5), FRFK (SEQ ID NO:6), FRFE (SEQ ID NO:7), FRFD (SEQ ID NO:8), FKFE (SEQ ID NO:9), FKFK (SEQ ID NO:10), FKFR (SEQ ID NO:11), FKFD (SEQ ID NO:12), FDFD (SEQ ID NO:13), FDFE (SEQ ID NO:14), FDFR (SEQ ID NO:15), FDFK (SEQ ID NO:16), WEWE (SEQ ID NO:17), WKWK (SEQ ID NO:18), WRWR (SEQ ID NO:19), WEWK (SEQ ID NO:20), WKWE (SEQ ID NO:21), WEWR (SEQ ID NO:22), WRWE (SEQ ID NO:23), WKWR (SEQ ID NO:24), WRWK (SEQ ID NO:25), WDWD (SEQ ID NO:26), WDWE (SEQ ID NO:27), WEWD (SEQ ID NO:28), WDWK (SEQ ID NO:29), WKWD (SEQ ID NO:30), WDWR (SEQ ID NO:31), WRWD (SEQ ID NO:32), IEIE (SEQ ID NO:33), IEIK (SEQ ID NO:34), IRIR (SEQ ID NO:35), IKIK (SEQ ID NO:36), IKIE (SEQ ID NO:37), IEIR (SEQ ID NO:38), IRIE (SEQ ID NO:39), IKIR (SEQ ID NO:40), IRIK (SEQ ID NO:41), IDID (SEQ ID NO:42), IDFE (SEQ ID NO:43), IEID (SEQ ID NO:44), IDIK (SEQ ID NO:45), IKID (SEQ ID NO:46), IDIR (SEQ ID NO:47), IRID (SEQ ID NO:48), YEYE (SEQ ID NO:49), YKYK (SEQ ID NO:50), YRYR (SEQ ID NO:51), YEYK (SEQ ID NO:52), YKYE (SEQ ID NO:53), YEYR (SEQ ID NO:54), YRYE (SEQ ID NO:55), YKYR (SEQ ID NO:56), YRYK (SEQ ID NO:57), YDYD (SEQ ID NO:58), YDYE (SEQ ID NO:59), YEYD (SEQ ID NO:60), YDYK (SEQ ID NO:61), YKYD (SEQ ID NO:62), YDYR (SEQ ID NO:63), YRYD (SEQ ID NO:64), Nle-E-Nle-E (SEQ ID NO:65), Nle-K-Nle-K (SEQ ID NO:66), Nle-R-Nle-R (SEQ ID NO:67), Nle-E-Nle-K (SEQ ID NO:68), Nle-K-Nle-E (SEQ ID NO:69), Nle-E-Nle-R (SEQ ID NO:70), Nle-R-Nle-E (SEQ ID NO:71), Nle-K-Nle-R (SEQ ID NO:72), Nle-R-Nle-K (SEQ ID NO:73), Nle-D-Nle-D (SEQ ID NO:74), Nle-D-Nle-E (SEQ ID NO:75), Nle-E-Nle-D (SEQ ID NO:76), Nle-D-Nle-K (SEQ ID NO:77), Nle-K-Nle-D (SEQ ID NO:78), Nle-D-Nle-R (SEQ ID NO:79), and Nle-R-Nle-D (SEQ ID NO:80).

6. The compound according to any one of the claims 1 to 4, wherein the peptide moiety is selected from the group consisting of FEFKFEFK (SEQ ID NO:81), FEFEFKFK (SEQ ID NO:82), FDFKFDFK (SEQ ID NO:83), FDFDFKFK (SEQ ID NO:84), FEFRFEFR (SEQ ID NO:85), FEFEFRFR (SEQ ID NO:86), YDYKYDYK (SEQ ID NO:87), YDYDYKYK (SEQ ID NO:88), YEYRYEYR (SEQ ID NO:89), YEYKYEYK (SEQ ID NO:90), YEYEYKYK (SEQ ID NO:91), WEWKWEWK (SEQ ID NO:92), WEWEWKWK (SEQ ID NO:93), WDWKWDWK (SEQ ID NO:94), WDWDWKWK (SEQ ID NO:95).

7. The compound according to any one of the claims 1 to 6, wherein the peptide moiety is FEFKFEFK (SEQ ID NO:81).

8. The compound according to any one of the claims 1 to 7, wherein the peptide moiety is covalently linked to the short-chain polyethylene glycol moiety through a terminus of the peptide moiety, preferably through the N-terminus of the peptide moiety.

9. A composition comprising the compound according to any one of the claims 1 to 8 and further comprising at least one solvent and/or at least one additive.

10. The composition according to claim 9 further comprising at least one further peptide.

11. A pharmaceutical composition comprising the compound according to any one of the claims 1 to 8.

12. The pharmaceutical composition according to claim 11 further comprising cells for cell therapy.

13. A hydrogel comprising the compound according to any one of the claims 1 to 8 or comprising the composition according to any one of the claims 9 to 12.

14. The compound according to any one of the claims 1 to 8 for use as a vehicle in cell therapy.

15. A method for the manufacture of the compound according to any one of the claims 1 to 8 comprising a peptide coupling step in solution.


SEQUENCE LISTING

<110>  Solvay SA

<120>  Peptides comprising a short-chain polyethylene glycol moiety

<130>  S 2012/061

<160>  106

<170>  PatentIn version 3.5


<210>  1
<211>  4
<212>  PRT
<213>  Artificial Sequence

<220>
<223>  Synthetic  peptide

<400>  1

Phe Glu Phe Glu
1


<210>  2
<211>  4
<212>  PRT
<213>  Artificial Sequence

<220>
<223>  Synthetic peptide

<400>  2

Phe Glu Phe Lys
1


<210>  3
<211>  4
<212>  PRT
<213>  Artificial Sequence

<220>
<223>  Synthetic peptide

<400>  3

Phe Glu Phe Asp
1


<210>  4
<211>  4
<212>  PRT
<213>  Artificial Sequence

<220>
<223>  Synthetic peptide

<400>  4

Phe Glu Phe Arg
1

<210>  5
<211>  4
<212>  PRT
<213>  Artificial Sequence

<220>
<223>  Synthetic peptide

<400>  5

Phe Arg Phe Arg
1


<210>  6
<211>  4
<212>  PRT
<213>  Artificial Sequence

<220>
<223>  Synthetic peptide

<400>  6

Phe Arg Phe Lys
1


<210>  7
<211>  4
<212>  PRT
<213>  Artificial Sequence

<220>
<223>  Synthetic peptide

<400>  7

Phe Arg Phe Glu
1


<210>  8
<211>  4
<212>  PRT
<213>  Artificial Sequence

<220>
<223>  Synthetic peptide

<400>  8

Phe Arg Phe Asp
1


<210>  9
<211>  4
<212>  PRT
<213>  Artificial Sequence

<220>
<223>  Synthetic peptide

<400>  9

Phe Lys Phe Glu
1

<210>  10
<211>  4
<212>  PRT
<213>  Artificial Sequence

<220>
<223>  Synthetic peptide

<400>  10

Phe Lys Phe Lys
1


<210>  11
<211>  4
<212>  PRT
<213>  Artificial Sequence

<220>
<223>  Synthetic peptide

<400>  11

Phe Lys Phe Arg
1


<210>  12
<211>  4
<212>  PRT
<213>  Artificial Sequence

<220>
<223>  Synthetic peptide

<400>  12

Phe Lys Phe Asp
1


<210>  13
<211>  4
<212>  PRT
<213>  Artificial Sequence

<220>
<223>  Synthetic peptide

<400>  13

Phe Asp Phe Asp
1


<210>  14
<211>  4
<212>  PRT
<213>  Artificial Sequence

<220>
<223>  Synthetic peptide

<400>  14

Phe Asp Phe Glu
1

<210>  15
<211>  4
<212>  PRT
<213>  Artificial Sequence

<220>
<223>  Synthetic peptide

<400>  15

Phe Asp Phe Arg
1


<210>  16
<211>  4
<212>  PRT
<213>  Artificial Sequence

<220>
<223>  Synthetic peptide

<400>  16

Phe Asp Phe Lys
1


<210>  17
<211>  4
<212>  PRT
<213>  Artificial Sequence

<220>
<223>  Synthetic peptide

<400>  17

Trp Glu Trp Glu
1


<210>  18
<211>  4
<212>  PRT
<213>  Artificial Sequence

<220>
<223>  Synthetic peptide

<400>  18

Trp Lys Trp Lys
1


<210>  19
<211>  4
<212>  PRT
<213>  Artificial Sequence

<220>
<223>  Synthetic peptide

<400>  19

Trp Arg Trp Arg

1

<210>  20
<211>  4
<212>  PRT
<213>  Artificial Sequence

<220>
<223>  Synthetic peptide

<400>  20

Trp Glu Trp Lys
1

<210>  21
<211>  4
<212>  PRT
<213>  Artificial Sequence

<220>
<223>  Synthetic peptide

<400>  21

Trp Lys Trp Glu
1

<210>  22
<211>  4
<212>  PRT
<213>  Artificial Sequence

<220>
<223>  Synthetic peptide

<400>  22

Trp Glu Trp Arg
1

<210>  23
<211>  4
<212>  PRT
<213>  Artificial Sequence

<220>
<223>  Synthetic peptide

<400>  23

Trp Arg Trp Glu
1

<210>  24
<211>  4
<212>  PRT
<213>  Artificial Sequence

<220>
<223>  Synthetic peptide

<400>  24

Trp Lys Trp Arg
1


<210>  25
<211>  4
<212>  PRT
<213>  Artificial Sequence

<220>
<223>  Synthetic peptide

<400>  25

Trp Arg Trp Lys
1


<210>  26
<211>  4
<212>  PRT
<213>  Artificial Sequence

<220>
<223>  Synthetic peptide

<400>  26

Trp Asp Trp Asp
1


<210>  27
<211>  4
<212>  PRT
<213>  Artificial Sequence

<220>
<223>  Synthetic peptide

<400>  27

Trp Asp Trp Glu
1


<210>  28
<211>  4
<212>  PRT
<213>  Artificial Sequence

<220>
<223>  Synthetic peptide

<400>  28

Trp Glu Trp Asp
1


<210>  29
<211>  4
<212>  PRT
<213>  Artificial Sequence

<220>
<223>  Synthetic peptide

<400>  29

Trp Asp Trp Lys
1



<210>  30
<211>  4
<212>  PRT
<213>  Artificial Sequence

<220>
<223>  Synthetic peptide

<400>  30

Trp Lys Trp Asp
1



<210>  31
<211>  4
<212>  PRT
<213>  Artificial Sequence

<220>
<223>  Synthetic peptide

<400>  31

Trp Asp Trp Arg
1



<210>  32
<211>  4
<212>  PRT
<213>  Artificial Sequence

<220>
<223>  Synthetic peptide

<400>  32

Trp Arg Trp Asp
1



<210>  33
<211>  4
<212>  PRT
<213>  Artificial Sequence

<220>
<223>  Synthetic peptide

<400>  33

Ile Glu Ile Glu
1



<210>  34
<211>  4
<212>  PRT
<213>  Artificial Sequence

<220>
<223>  Synthetic peptide

<400>  34

Ile Glu Ile Lys
1


<210>  35
<211>  4
<212>  PRT
<213>  Artificial Sequence

<220>
<223>  Synthetic peptide

<400>  35

Ile Arg Ile Arg
1


<210>  36
<211>  4
<212>  PRT
<213>  Artificial Sequence

<220>
<223>  Synthetic peptide

<400>  36

Ile Lys Ile Lys
1


<210>  37
<211>  4
<212>  PRT
<213>  Artificial Sequence

<220>
<223>  Synthetic peptide

<400>  37

Ile Lys Ile Glu
1


<210>  38
<211>  4
<212>  PRT
<213>  Artificial Sequence

<220>
<223>  Synthetic peptide

<400>  38

Ile Glu Ile Arg
1


<210>  39
<211>  4
<212>  PRT
<213>  Artificial Sequence

<220>
<223>  Synthetic peptide

<400>  39

Ile Arg Ile Glu
1


<210>  40
<211>  4
<212>  PRT
<213>  Artificial Sequence

<220>
<223>  Synthetic peptide

<400>  40

Ile Lys Ile Arg
1


<210>  41
<211>  4
<212>  PRT
<213>  Artificial Sequence

<220>
<223>  Synthetic peptide

<400>  41

Ile Arg Ile Lys
1


<210>  42
<211>  4
<212>  PRT
<213>  Artificial Sequence

<220>
<223>  Synthetic peptide

<400>  42

Ile Asp Ile Asp
1


<210>  43
<211>  4
<212>  PRT
<213>  Artificial Sequence

<220>
<223>  Synthetic peptide

<400>  43

Ile Asp Phe Glu
1


<210>  44
<211>  4
<212>  PRT
<213>  Artificial Sequence

<220>

<223>  Synthetic peptide

<400>  44

Ile Glu Ile Asp
1


<210>  45
<211>  4
<212>  PRT
<213>  Artificial Sequence

<220>
<223>  Synthetic peptide

<400>  45

Ile Asp Ile Lys
1


<210>  46
<211>  4
<212>  PRT
<213>  Artificial Sequence

<220>
<223>  Synthetic peptide

<400>  46

Ile Lys Ile Asp
1


<210>  47
<211>  4
<212>  PRT
<213>  Artificial Sequence

<220>
<223>  Synthetic peptide

<400>  47

Ile Asp Ile Arg
1


<210>  48
<211>  4
<212>  PRT
<213>  Artificial Sequence

<220>
<223>  Synthetic peptide

<400>  48

Ile Arg Ile Asp
1


<210>  49
<211>  4
<212>  PRT
<213>  Artificial Sequence

<220>
<223>  Synthetic peptide

<400>  49

Tyr Glu Tyr Glu
1


<210>  50
<211>  4
<212>  PRT
<213>  Artificial Sequence

<220>
<223>  Synthetic peptide

<400>  50

Tyr Lys Tyr Lys
1


<210>  51
<211>  4
<212>  PRT
<213>  Artificial Sequence

<220>
<223>  Synthetic peptide

<400>  51

Tyr Arg Tyr Arg
1


<210>  52
<211>  4
<212>  PRT
<213>  Artificial Sequence

<220>
<223>  Synthetic peptide

<400>  52

Tyr Glu Tyr Lys
1


<210>  53
<211>  4
<212>  PRT
<213>  Artificial Sequence

<220>
<223>  Synthetic peptide

<400>  53

Tyr Lys Tyr Glu
1


<210>  54
<211>  4
<212>  PRT
<213>  Artificial Sequence

<220>
<223>  Synthetic peptide

<400>  54

Tyr Glu Tyr Arg
1


<210>  55
<211>  4
<212>  PRT
<213>  Artificial Sequence

<220>
<223>  Synthetic peptide

<400>  55

Tyr Arg Tyr Glu
1


<210>  56
<211>  4
<212>  PRT
<213>  Artificial Sequence

<220>
<223>  Synthetic peptide

<400>  56

Tyr Lys Tyr Arg
1


<210>  57
<211>  4
<212>  PRT
<213>  Artificial Sequence

<220>
<223>  Synthetic peptide

<400>  57

Tyr Arg Tyr Lys
1


<210>  58
<211>  4
<212>  PRT
<213>  Artificial Sequence

<220>
<223>  Synthetic peptide

<400>  58

Tyr Asp Tyr Asp
1


<210>  59
<211>  4
<212>  PRT

<213>  Artificial Sequence

<220>
<223>  Synthetic peptide

<400>  59

Tyr Asp Tyr Glu
1


<210>  60
<211>  4
<212>  PRT
<213>  Artificial Sequence

<220>
<223>  Synthetic peptide

<400>  60

Tyr Glu Tyr Asp
1


<210>  61
<211>  4
<212>  PRT
<213>  Artificial Sequence

<220>
<223>  Synthetic peptide

<400>  61

Tyr Asp Tyr Lys
1


<210>  62
<211>  4
<212>  PRT
<213>  Artificial Sequence

<220>
<223>  Synthetic peptide

<400>  62

Tyr Lys Tyr Asp
1


<210>  63
<211>  4
<212>  PRT
<213>  Artificial Sequence

<220>
<223>  Synthetic peptide

<400>  63

Tyr Asp Tyr Arg
1


<210>  64
<211>  4

<212>  PRT
<213>  Artificial Sequence

<220>
<223>  Synthetic peptide

<400>  64

Tyr Arg Tyr Asp
1


<210>  65
<211>  4
<212>  PRT
<213>  Artificial Sequence

<220>
<223>  Synthetic peptide


<220>
<221>  VARIANT
<222>  (1)..(1)
<223>  Norleucine

<220>
<221>  VARIANT
<222>  (3)..(3)
<223>  Norleucine

<400>  65

Xaa Glu Xaa Glu
1


<210>  66
<211>  4
<212>  PRT
<213>  Artificial Sequence

<220>
<223>  Synthetic peptide


<220>
<221>  VARIANT
<222>  (1)..(1)
<223>  Norleucine

<220>
<221>  VARIANT
<222>  (3)..(3)
<223>  Norleucine

<400>  66

Xaa Lys Xaa Lys
1


<210>  67
<211>  4
<212>  PRT
<213>  Artificial Sequence

<220>
<223>  Synthetic peptide

<220>
<221>  VARIANT
<222>  (1)..(1)

<220>
<221>  VARIANT
<222>  (1)..(1)
<223>  Norleucine

<220>
<221>  VARIANT
<222>  (3)..(3)
<223>  Norleucine

<400>  67

Xaa Arg Xaa Arg
1


<210>  68
<211>  4
<212>  PRT
<213>  Artificial Sequence

<220>
<223>  Synthetic peptide


<220>
<221>  VARIANT
<222>  (1)..(1)
<223>  Norleucine

<220>
<221>  VARIANT
<222>  (3)..(3)
<223>  Norleucine

<400>  68

Xaa Glu Xaa Lys
1


<210>  69
<211>  4
<212>  PRT
<213>  Artificial Sequence

<220>
<223>  Synthetic peptide


<220>
<221>  VARIANT
<222>  (1)..(1)
<223>  Norleucine

<220>
<221>  VARIANT
<222>  (3)..(3)
<223>  Norleucine

<400>  69

Xaa Lys Xaa Glu

1



<210>  70
<211>  4
<212>  PRT
<213>  Artificial Sequence

<220>
<223>  Synthetic peptide


<220>
<221>  VARIANT
<222>  (1)..(1)
<223>  Norleucine

<220>
<221>  VARIANT
<222>  (3)..(3)
<223>  Norleucine

<400>  70

Xaa Glu Xaa Arg
1


<210>  71
<211>  4
<212>  PRT
<213>  Artificial Sequence

<220>
<223>  Synthetic peptide


<220>
<221>  VARIANT
<222>  (1)..(1)
<223>  Norleucine

<220>
<221>  VARIANT
<222>  (3)..(3)
<223>  Norleucine

<400>  71

Xaa Arg Xaa Glu
1


<210>  72
<211>  4
<212>  PRT
<213>  Artificial Sequence

<220>
<223>  Synthetic peptide


<220>
<221>  VARIANT
<222>  (1)..(1)
<223>  Norleucine

<220>
<221>  VARIANT

<222>  (3)..(3)
<223>  Norleucine

<400>  72

Xaa Lys Xaa Arg
1


<210>  73
<211>  4
<212>  PRT
<213>  Artificial Sequence

<220>
<223>  Synthetic peptide


<220>
<221>  VARIANT
<222>  (1)..(1)
<223>  Norleucine

<220>
<221>  VARIANT
<222>  (3)..(3)
<223>  Norleucine

<400>  73

Xaa Arg Xaa Lys
1


<210>  74
<211>  4
<212>  PRT
<213>  Artificial Sequence

<220>
<223>  Synthetic peptide


<220>
<221>  VARIANT
<222>  (1)..(1)
<223>  Norleucine

<220>
<221>  VARIANT
<222>  (3)..(3)
<223>  Norleucine

<400>  74

Xaa Asp Xaa Asp
1


<210>  75
<211>  4
<212>  PRT
<213>  Artificial Sequence

<220>
<223>  Synthetic peptide


<220>

<221>  VARIANT
<222>  (1)..(1)

<220>
<221>  VARIANT
<222>  (1)..(1)
<223>  Norleucine

<220>
<221>  VARIANT
<222>  (3)..(3)
<223>  Norleucine

<400>  75

Xaa Asp Xaa Glu
1


<210>  76
<211>  4
<212>  PRT
<213>  Artificial Sequence

<220>
<223>  Synthetic peptide


<220>
<221>  VARIANT
<222>  (1)..(1)
<223>  Norleucine

<220>
<221>  VARIANT
<222>  (3)..(3)
<223>  Norleucine

<400>  76

Xaa Glu Xaa Asp
1


<210>  77
<211>  4
<212>  PRT
<213>  Artificial Sequence

<220>
<223>  Synthetic peptide


<220>
<221>  VARIANT
<222>  (1)..(1)
<223>  Norleucine

<220>
<221>  VARIANT
<222>  (3)..(3)
<223>  Norleucine

<400>  77

Xaa Asp Xaa Lys
1

<210>  78
<211>  4
<212>  PRT
<213>  Artificial Sequence

<220>
<223>  Synthetic peptide


<220>
<221>  VARIANT
<222>  (1)..(1)
<223>  Norleucine

<220>
<221>  VARIANT
<222>  (3)..(3)
<223>  Norleucine

<400>  78

Xaa Lys Xaa Asp
1


<210>  79
<211>  4
<212>  PRT
<213>  Artificial Sequence

<220>
<223>  Synthetic peptide


<220>
<221>  VARIANT
<222>  (1)..(1)
<223>  Norleucine

<220>
<221>  VARIANT
<222>  (3)..(3)
<223>  Norleucine

<400>  79

Xaa Asp Xaa Arg
1


<210>  80
<211>  4
<212>  PRT
<213>  Artificial Sequence

<220>
<223>  Synthetic peptide


<220>
<221>  VARIANT
<222>  (1)..(1)
<223>  Norleucine

<220>
<221>  VARIANT
<222>  (3)..(3)
<223>  Norleucine

<400>  80

Xaa Arg Xaa Asp
1


<210>  81
<211>  8
<212>  PRT
<213>  Artificial Sequence

<220>
<223>  Synthetic peptide

<400>  81

Phe Glu Phe Lys Phe Glu Phe Lys
1               5


<210>  82
<211>  8
<212>  PRT
<213>  Artificial Sequence

<220>
<223>  Synthetic peptide

<400>  82

Phe Glu Phe Glu Phe Lys Phe Lys
1               5


<210>  83
<211>  8
<212>  PRT
<213>  Artificial

<220>
<223>  Synthetic peptide

<400>  83

Phe Asp Phe Lys Phe Asp Phe Lys
1               5


<210>  84
<211>  8
<212>  PRT
<213>  Artificial

<220>
<223>  Synthetic peptide

<400>  84

Phe Asp Phe Asp Phe Lys Phe Lys
1               5


<210>  85
<211>  8
<212>  PRT
<213>  Artificial

<220>
<223>  Synthetic peptide

<400>  85

Phe Glu Phe Arg Phe Glu Phe Arg
1               5


<210>  86
<211>  8
<212>  PRT
<213>  Artificial

<220>
<223>  Synthetic peptide

<400>  86

Phe Glu Phe Glu Phe Arg Phe Arg
1               5


<210>  87
<211>  8
<212>  PRT
<213>  Artificial

<220>
<223>  Synthetic peptide

<400>  87

Tyr Asp Tyr Lys Tyr Asp Tyr Lys
1               5


<210>  88
<211>  8
<212>  PRT
<213>  Artificial

<220>
<223>  Synthetic peptide

<400>  88

Tyr Asp Tyr Asp Tyr Lys Tyr Lys
1               5


<210>  89
<211>  8
<212>  PRT
<213>  Artificial

<220>
<223>  Synthetic peptide

<400>  89

Tyr Glu Tyr Arg Tyr Glu Tyr Arg
1               5


<210>  90
<211>  8
<212>  PRT
<213>  Artificial

<220>

<223>  Synthetic peptide

<400>  90

Tyr Glu Tyr Lys Tyr Glu Tyr Lys
1               5


<210>  91
<211>  8
<212>  PRT
<213>  Artificial

<220>
<223>  Synthetic peptide

<400>  91

Tyr Glu Tyr Glu Tyr Lys Tyr Lys
1               5


<210>  92
<211>  8
<212>  PRT
<213>  Artificial

<220>
<223>  Synthetic peptide

<400>  92

Trp Glu Trp Lys Trp Glu Trp Lys
1               5


<210>  93
<211>  8
<212>  PRT
<213>  Artificial

<220>
<223>  Synthetic peptide

<400>  93

Trp Glu Trp Glu Trp Lys Trp Lys
1               5


<210>  94
<211>  8
<212>  PRT
<213>  Artificial

<220>
<223>  Synthetic peptide

<400>  94

Trp Asp Trp Lys Trp Asp Trp Lys
1               5


<210>  95
<211>  8
<212>  PRT
<213>  Artificial

<220>
<223>  Synthetic peptide

<400>  95

Trp Asp Trp Asp Trp Lys Trp Lys
1               5

<210>  96
<211>  8
<212>  PRT
<213>  Artificial Sequence

<220>
<223>  Synthetic peptide

<220>
<221>  VARIANT
<222>  (1)..(1)
<223>  carboxybenzyl phenylalanine

<220>
<221>  VARIANT
<222>  (2)..(2)
<223>  O-tert-butyl glutamic acid

<220>
<221>  VARIANT
<222>  (4)..(4)
<223>  N(6)-benzyloxycarbonyl lycine

<220>
<221>  VARIANT
<222>  (6)..(6)
<223>  O-tert-butyl glutamic acid

<220>
<221>  VARIANT
<222>  (8)..(8)
<223>  N(6)-benzyloxycarbonyl lysine

<400>  96

Phe Glu Phe Lys Phe Glu Phe Lys
1               5

<210>  97
<211>  8
<212>  PRT
<213>  Artificial Sequence

<220>
<223>  Synthetic peptide

<220>
<221>  VARIANT
<222>  (1)..(1)
<223>  Carboxybenzyl phenylalanine

<220>
<221>  VARIANT
<222>  (2)..(2)
<223>  O-tert-butyl glutamic acid

<220>
<221>  VARIANT
<222>  (4)..(4)
<223>  N(6)-benzyloxycarbonyl lysine

<220>
<221>  VARIANT
<222>  (6)..(6)
<223>  O-tert-butyl glutamic acid

<220>
<221>  VARIANT
<222>  (8)..(8)
<223>  ethyl N(6)-benzyloxycarbonyl lysinate

<400>  97

Phe Glu Phe Lys Phe Glu Phe Lys
1               5


<210>  98
<211>  8
<212>  PRT
<213>  Artificial Sequence

<220>
<223>  Synthetic peptide


<220>
<221>  VARIANT
<222>  (2)..(2)
<223>  O-tert-butyl glutamic acid

<220>
<221>  VARIANT
<222>  (4)..(4)
<223>  N(6)-benzyloxycarbonyl lysine

<220>
<221>  VARIANT
<222>  (6)..(6)
<223>  O-tert-butyl glutamic acid

<220>
<221>  VARIANT
<222>  (8)..(8)
<223>  ethyl N(6)-benzyloxycarbonyl lysinate

<400>  98

Phe Glu Phe Lys Phe Glu Phe Lys
1               5


<210>  99
<211>  8
<212>  PRT
<213>  Artificial Sequence

<220>
<223>  Synthetic peptide


<220>
<221>  VARIANT
<222>  (1)..(1)
<223>  BenzyloxycarbonylHNCH2CH2OCH2CH2OCH2C(O)-phenylalanine

<220>
<221>  VARIANT
<222>  (2)..(2)
<223>  O-tert-butyl glutamic acid

<220>
<221>  VARIANT
<222>  (4)..(4)
<223>  N(6)-benzyloxycarbonyl lysine

<220>
<221>  VARIANT
<222>  (6)..(6)
<223>  O-tert-butyl glutamic acid

<220>
<221>  VARIANT
<222>  (8)..(8)
<223>  ethyl N(6)-benzyloxycarbonyl lysinate

<400>  99

Phe Glu Phe Lys Phe Glu Phe Lys
1               5


<210>  100
<211>  8
<212>  PRT
<213>  Artificial Sequence

<220>
<223>  Synthetic peptide


<220>
<221>  VARIANT
<222>  (1)..(1)
<223>  H2NCH2CH2OCH2CH2OCH2C(O)-phenylalanine

<220>
<221>  VARIANT
<222>  (8)..(8)
<223>  ethyl N(6)-benzyloxycarbonyl lysinate

<400>  100

Phe Glu Phe Lys Phe Glu Phe Lys
1               5


<210>  101
<211>  8
<212>  PRT
<213>  Artificial Sequence

<220>
<223>  Synthetic peptide


<220>
<221>  VARIANT
<222>  (1)..(1)
<223>  HOOC-CH2-O-CH2-C(O)-phenylalanine

<220>
<221>  VARIANT
<222>  (2)..(2)

<223>  O-tert-butyl glutamic acid

<220>
<221>  VARIANT
<222>  (4)..(4)
<223>  N(6)-benzyloxycarbonyl lysine

<220>
<221>  VARIANT
<222>  (6)..(6)
<223>  O-tert-butyl glutamic acid

<220>
<221>  VARIANT
<222>  (8)..(8)
<223>  ethyl N(6)-benzyloxycarbonyl lysinate

<400>  101

Phe Glu Phe Lys Phe Glu Phe Lys
1               5


<210>  102
<211>  8
<212>  PRT
<213>  Artificial Sequence

<220>
<223>  Synthetic peptide


<220>
<221>  VARIANT
<222>  (1)..(1)
<223>  HOOCCH2OCH2CH2OCH2CH2NC(O)CH2OCH2C(O)-phenylalanine

<220>
<221>  VARIANT
<222>  (2)..(2)
<223>  O-tert-butyl glutamic acid

<220>
<221>  VARIANT
<222>  (4)..(4)
<223>  N(6)-benzyloxycarbonyl lysine

<220>
<221>  VARIANT
<222>  (6)..(6)
<223>  O-tert-butyl glutamic acid

<220>
<221>  VARIANT
<222>  (8)..(8)
<223>  ethyl N(6)-benzyloxycarbonyl lysinate

<400>  102

Phe Glu Phe Lys Phe Glu Phe Lys
1               5


<210>  103
<211>  8
<212>  PRT
<213>  Artificial Sequence

<220>

<223>  Synthetic peptide


<220>
<221>  VARIANT
<222>  (1)..(1)
<223>  HOOCCH2OCH2CH2OCH2CH2NC(O)CH2OCH2C(O)-phenylalanine

<220>
<221>  VARIANT
<222>  (8)..(8)
<223>  ethyl N(6)-benzyloxycarbonyl lysinate

<400>  103

Phe Glu Phe Lys Phe Glu Phe Lys
1               5


<210>  104
<211>  8
<212>  PRT
<213>  Artificial Sequence

<220>
<223>  Synthetic peptide


<220>
<221>  VARIANT
<222>  (1)..(1)
<223>  carboxybenzyl phenylalanine

<220>
<221>  VARIANT
<222>  (2)..(2)
<223>  O-tert-butyl glutamic acid

<220>
<221>  VARIANT
<222>  (4)..(4)
<223>  N(6)-benzyloxycarbonyl lysine

<220>
<221>  VARIANT
<222>  (6)..(6)
<223>  O-tert-butyl glutamic acid

<220>
<221>  VARIANT
<222>  (8)..(8)
<223>  N(6)-benzyloxycarbonyl
       lysine-NH-(CH2)3-O-(CH2)2-O-(CH2)2-O-(CH2)3-NH-benyzloxycarbonyl

<400>  104

Phe Glu Phe Lys Phe Glu Phe Lys
1               5


<210>  105
<211>  8
<212>  PRT
<213>  Artificial Sequence

<220>
<223>  Synthetic peptide

<220>
<221>  VARIANT
<222>  (2)..(2)
<223>  O-tert-butyl glutamic acid

<220>
<221>  VARIANT
<222>  (4)..(4)
<223>  N(6)-benzyloxycarbonyl lysine

<220>
<221>  VARIANT
<222>  (6)..(6)
<223>  O-tert-butyl glutamic acid

<220>
<221>  VARIANT
<222>  (8)..(8)
<223>  N(6)-benzyloxycarbonyl
       lysine-NH-(CH2)3-O-(CH2)2-O-(CH2)2-O-(CH2)3-NH-benyzloxycarbonyl

<400>  105

Phe Glu Phe Lys Phe Glu Phe Lys
1               5


<210>  106
<211>  8
<212>  PRT
<213>  Artificial Sequence

<220>
<223>  Synthetic peptide


<220>
<221>  VARIANT
<222>  (8)..(8)
<223>  lysine-NH-(CH2)3-O-(CH2)2-O-(CH2)2-O-(CH2)3-NH2

<400>  106

Phe Glu Phe Lys Phe Glu Phe Lys
1               5